# EUROPÄISCHE PATENTSCHRIFT

(11) **EP 2 582 673 B1**
(45) Veröffentlichungstag und Bekanntmachung des Hinweises auf die Patenterteilung: **06.08.2014**
(21) Anmeldenummer: 11724248.7
(22) Anmeldetag: 10.06.2011
(51) Int. Cl.: C07D 213/61

(54) **VERFAHREN ZUR HERSTELLUNG VON 2,2-DIFLUORETHYLAMIN-DERIVATEN DURCH ALKYLIERUNG MIT 2,2- DIFLUOR-1-HALOGENETHANEN**
PROCESS FOR THE PREPARATION OF 2,2-DIFLUOROETHYLAMINE DERIVATIVES BY ALKYLATION WITH 2,2-DIFLUORO-1-HALOGENO-ETHANES
.PROCÉDÉ DE PRÉPARATION DE DÉRIVÉS DE 2,2-DIFLUORÉTHYLAMINE PAR ALKYLATION AVEC 2,2-DIFLUORO-1-HALOGÉNO-ÉTHANES

(30) Priorität: 15.06.2010 US 354933 P; 15.06.2010 EP 10166019
(43) Veröffentlichungstag der Anmeldung: 24.04.2013
(73) Patentinhaber: Bayer Intellectual Property GmbH, 40789 Monheim am Rhein (DE)
(72) Erfinder: LUI, Norbert, 51519 Odenthal (DE); HEINRICH, Jens-Dietmar, 51381 Leverkusen (DE); MORADI, Wahed, Ahmed, 40789 Monheim (DE); FUNKE, Christian, 42799 Leichlingen (DE)
(74) Vertreter: Weismantel, Lothar
(86) Internationale Anmeldenummer: PCT/EP2011/059691
(87) Internationale Veröffentlichungsnummer: WO 2011/157650

(56) Entgegenhaltungen:
- WO-A1-2007/115644
- US-A1- 2006 014 764

## Beschreibung

Die vorliegende Erfindung betrifft ein Verfahren zur Herstellung bestimmter 2,2-Difluorethylamin-Derivate ausgehend von 2,2-Difluorethyl-1-halogenethan.

2,2-Difluorethylamin-Derivate sind nützliche Zwischenstufen bei der Herstellung agrochemischer Wirkstoffe (siehe WO 2007/115644). Es sind verschiedene Verfahren zur Herstellung von 2,2-Difluorethylamin-Derivaten bekannt.

Die WO 2009/036900 beschreibt beispielsweise ein Verfahren zur Herstellung von 2,2-Difluorethylamin-Derivaten durch Amid-Hydrierung von N-[(6-Chlorpyridin-3-yl)methyl]-2,2-difluoracetamid (Schema 1).

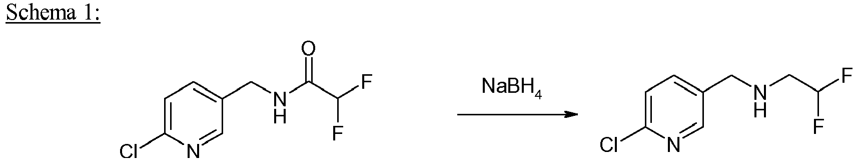

Nachteilig bei diesem Verfahren ist der Einsatz komplexer Hydride wie Natriumborhydrid, weil Hydride teuer und sicherheitstechnisch aufwendig zu verwenden sind.

WO 2009/036901 beschreibt die Reduktion von N-(6-Chlorpyridin-3-yl)methylen-2,2-difluorethanamin durch Wasserstoff (Schema 2).

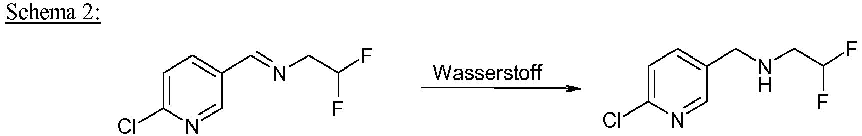

Nachteilig bei diesem Verfahren ist der Einsatz von Wasserstoff, weil auch hier die Verwendung von Wasserstoff sicherheitstechnisch sehr aufwendig ist.

Die Druckschrift WO 2007/115644, die sich mit der Herstellung von insektizid wirksamen 4-Aminobut-2-enolidverbindungen befasst, beschreibt die Herstellung von Verbindungen der allgemeinen Formel A-CH₂-NH-R¹, in der A für spezielle Heterocyclen und R¹ für Halogenalkyl steht, durch Alkylierung des Stickstoffs (Schema 3).

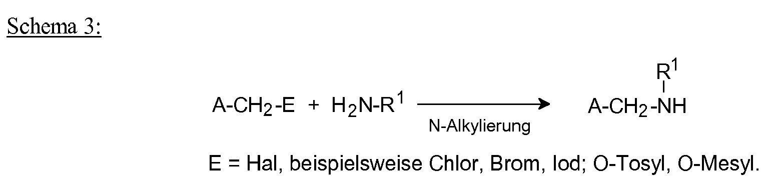

Konkret beschreibt WO2007/115644 die Herstellung von N-[(6-Chlorpyridin-3-yl)methyl]-2,2-difluorethan-1-amin (Verbindung (3)), das ausgehend von 2-Chlor-5-chlormethyl-pyridin (Verbindung (2)) und 2,2-Difluorethan-1-amin (Verbindung (1)) in Gegenwart von Triethylamin synthetisiert wird (siehe Schema 4). Die Verbindungen (1), (2) und Triethylamin werden dabei in äquimolaren Mengen eingesetzt. Das gewünschte Produkt wird in einer Ausbeute von 53 % erhalten.

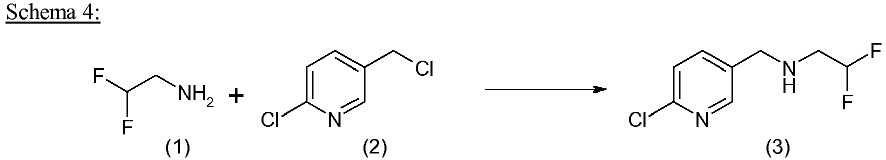

WO 2007/115644 beschreibt ferner, dass die Verbindungen N-[(6-Chlorpyridin-3-yl)methyl]-3-fluor-propan-1-amin, und N-[(6-Chlorpyridin-3-yl)methyl]-2-chlor-2-fluorethan-1-amin in gleicher Weise hergestellt wurden.

Das in WO 2007/116544 beschriebene Verfahren zur Herstellung von Verbindungen der Formel A-CH₂-NH-R¹, in der A für spezielle Heterocyclen und R¹ für Halogenalkyl steht, ist nachteilig, da es während der Umsetzung zu Mehrfachalkylierungen des Stickstoffs kommen kann. Dies führt zu einem Ausbeuteverlust, was auch an der Ausbeute des konkret genannten Beispiels zu erkennen ist. Die Ausbeute betrug lediglich 53 %. Diese Mehrfachalkylierungen können nur durch den Einsatz eines großen Überschusses an Amin reduziert werden. Abgesehen davon, dass Amine oft sehr kostenintensiv sind, ist das Verfahren auch deshalb unwirtschaftlich, da das im Überschuss zugegebene und nicht umgesetzte Amin entweder entsorgt oder aufwändig zurück gewonnen werden muss.

Wegen der Bedeutung von 2,2-Difluorethylamin-Derivaten als Bausteine zur Synthese agrochemischer Wirkstoffe ist es jedoch notwendig ein Verfahren zu finden, das großtechnisch und kostengünstig eingesetzt werden kann. Auch ist es erstrebenswert die speziellen 2,2-Difluorethylamin-Derivate mit hoher Ausbeute und hoher Reinheit zu erhalten, so dass die Zielverbindung vorzugsweise keiner weiteren möglicherweise komplexen Aufreinigung unterzogen werden muss. Die oben genannten Verfahren sind jedoch nicht dazu geeignet.

Es wurde nun ein Verfahren zur Herstellung bestimmter 2,2-Difluorethylamin-Derivate gefunden, das die Nachteile der bekannten Verfahren vermeidet und überdies hinaus noch einfach und kostengünstig durchzuführen ist, sodass es großtechnisch eingesetzt werden kann.

Die vorliegende Erfindung betrifft somit ein Verfahren zur Herstellung bestimmter 2,2-Difluorethylamin-Derivate der allgemeinen Formel (III) 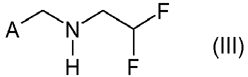 in der
- A: für einen gegebenenfalls substituierten Heterocyclus steht, der ausgewählt ist aus einer Gruppe bestehend aus Pyrid-2-yl, Pyrid-4-yl und Pyrid-3-yl, die gegebenenfalls in 6-Position substituiert sind durch Fluor, Chlor, Brom, Methyl, Trifluormethyl oder Trifluormethoxy, sowie Pyridazin-3-yl, welches gegebenenfalls in 6-Position substituiert ist durch Chlor oder Methyl sowie Pyrazin-3-yl, 2-Chlor-pyrazin-5-yl und 1,3-Thiazol-5-yl, wobei 1,3-Thiazol-5-yl gegebenenfalls in 2-Position substituiert ist durch Chlor oder Methyl, sowie Pyrimidinyl, Pyrazolyl, Thiophenyl, Oxazolyl, Isoxazolyl, 1,2,4-Oxadiazolyl, Isothiazolyl, 1,2,4-Triazolyl und 1,2,5-Thiadiazolyl, die gegebenenfalls durch Fluor, Chlor, Brom, Cyano, Nitro, gegebenenfalls durch Fluor und/oder Chlor substituiertes C₁-C₄-Alkyl, gegebenenfalls durch Fluor und/oder Chlor substituiertes C₁-C₃-Alkylthio, oder gegebenenfalls durch Fluor und/oder Chlor substituiertes C₁-C₃-Alkylsulfonyl substituiert sind sowie Pyrid-3-yl der folgenden Formel 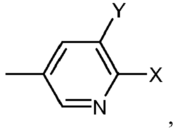 in der
- X: für Halogen, C₁-C₁₂-Alkyl oder C₁-C₁₂-Halogenalkyl steht und
- Y: für Halogen, C₁-C₁₂-Alkyl, C₁-C₁₂-Halogenalkyl, C₁-C₁₂-Halogenalkoxy, Azido oder Cyan steht,
in dem eine 2,2-Difluorethyl-1-halogenethan Verbindung der allgemeinen Formel (I) 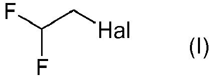 in der Hal für Cl, Br oder Jod steht
mit einem Amin der allgemeinen Formel (II) 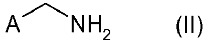 in der A die vorgenannten Bedeutungen hat, gegebenenfalls in Gegenwart einer Base umgesetzt wird.

Die erfindungsgemäße Reaktion ist in Schema 5 dargestellt.

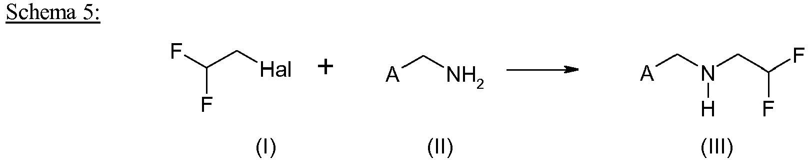

Die gewünschten 2,2-Difluorethylamin-Derivate der allgemeinen Formel (III) werden mit dem erfindungsgemäßen Verfahren mit guten Ausbeuten und in hoher Reinheit erhalten. Die gewünschten Verbindungen werden dabei in einer Reinheit erhalten, welche eine umfangreiche Aufarbeitung des Reaktionsprodukts im Allgemeinen nicht erforderlich macht.

Mit dem erfindungsgemäßen Verfahren können bessere Ausbeuten erzielt werden als mit dem in WO 2007/115644 beschriebenen Verfahren.

Im Rahmen der vorliegenden Erfindung wird unter einem Derivat ein von dem bezeichneten organischen Grundgerüst (Baustein) abgeleiteter Stoff ähnlicher Struktur bezeichnet, d.h. unter einem 2,2-Difluorethylamin-Derivat wird insbesondere eine Verbindung verstanden, welche einen 2,2-Difluorethylamin-Baustein umfasst.

Bevorzugt wird eine 2,2-Difluor-1-halogenethan Verbindung der allgemeinen Formel (I) in der Hal für Chlor und Brom steht, eingesetzt. Besonders bevorzugt ist die Verbindung CHF₂-CH₂Cl (2,2-Difluor-1-chlorethan).

Weiterhin bevorzugt werden Verbindungen der Formel (II) im erfindungsgemäßen Verfahren eingesetzt, in der der Rest A ausgewählt ist aus einer Gruppe bestehend aus 6-Fluor-pyrid-3-yl, 6-Chlor-pyrid-3-yl, 6-Brom-pyrid-3-yl, 6-Methyl-pyrid-3-yl, 6-Trifluormethyl-pyrid-3-yl, 6-Trifluormethoxypyrid-3-yl, 6-Chlor-1,4-pyridazin-3-yl, 6-Methyl-1,4-pyridazin-3-yl, 2-Chlor-1,3-thiazol-5-yl oder 2-Methyl-l,3-thiazol-5-yl, 2-Chlor-pyrimidin-5-yl, 2-Trifluormethyl-pyrimidin-5-yl, 5,6-Difluor-pyrid-3-yl, 5-Chlor-6-fluor-pyrid-3-yl, 5-Brom-6-fluor-pyrid-3-yl, 5-Iod-6-fluor-pyrid-3-yl, 5-Fluor-6-chlor-pyrid-3-yl, 5,6-dichlor-pyrid-3-yl, 5-Brom-6-chlor-pyrid-3-yl, 5-Iod-6-chlor-pyrid-3-yl, 5-Fluor-6-brom-pyrid-3-yl, 5-Chlor-6-brom-pyrid-3-yl, 5,6-Dibrom-pyrid-3-yl, 5-Fluor-6-iod-pyrid-3-yl, 5-Chlor-6-iod-pyrid-3-yl, 5-Brom-6-iod-pyrid-3-yl, 5-Methyl-6-fluor-pyrid-3-yl, 5-Methyl-6-chlor-pyrid-3-yl, 5-Methyl-6-brom-pyrid-3-yl, 5-Methyl-6-iod-pyrid-3-yl, 5-Difluormethyl-6-fluor-pyrid-3-yl, 5-Difluormethyl-6-chlor-pyrid-3-yl, 5-Difluormethyl-6-brom-pyrid-3-yl und 5-Difluormethyl-6-iod-pyrid-3-yl. Bevorzugte Reste A sind 6-Fluor-pyrid-3-yl, 6-Chlor-pyrid-3-yl, 6-Brompyrid-3-yl, 6-Chlor-1,4-pyridazin-3-yl, 2-Chlor-1,3-thiazol-5-yl, 2-Chlor-pyrimidin-5-yl, 5-Fluor-6-chlor-pyrid-3-yl, 5,6-Dichlor-pyrid-3-yl, 5-Brom-6-chlor-pyrid-3-yl, 5-Fluor-6-brom-pyrid-3-yl, 5-Chlor-6-brom-pyrid-3-yl, 5,6-Dibrom-pyrid-3-yl, 5-Methyl-6-chlor-pyrid-3-yl, 5-Chlor-6-iod-pyrid-3-yl und 5-Difluormethyl-6-chlor-pyrid-3-yl. Besonders bevorzugte Reste A sind 6-Chlor-pyrid-3-yl, 6-Brom-pyrid-3-yl, 6-Chlor-1,4-pyridazin-3-yl, 2-Chlor-1,3-thiazol-5-yl, 5-Fluor-6-chlor-pyrid-3-yl und 5-Fluor-6-brom-pyrid-3-yl.

Das erfindungsgemäße Verfahren erfolgt vorzugsweise in Gegenwart einer Base. Das eingesetzte Amin der allgemeinen Formel (II) kann auch als Base fungieren. Entsprechend muss dann der Anteil an Amin der allgemeinen Formel (II) erhöht werden.

Erfindungsgemäß geeignete Basen sind beispielsweise tertiäre Stickstoffbasen wie tertiäre Amine, substituierte oder unsubstituierte Pyridine, substituierte oder unsubstituierte Chinoline, substituierte oder unsubstituierte Imidazole, Alkali- oder Erdalkalimetallhydroxyde, -hydrogencarbonate oder -carbonate sowie sonstige anorganische wässrige Basen.

Vorzugsweise werden substituierte oder unsubstituierte Pyridine, substituierte oder unsubstituierte Chinoline und tertiäre Amine der allgemeinen Formel (IV)

NR¹R²R³ (IV)

eingesetzt, in welcher
R¹, R² und R³ unabhängig voneinander für C₁₋₁₂-Alkyl, C₆₋₁₈-Aryl, C₇₋₁₉-Alkylaryl- oder C₇₋₁₉-Arylalkyl stehen, oder in der zwei der Reste zusammen für einen 5- bis 8- gliedrigen Stickstoff enthaltenden Heterocylus stehen, oder in der alle drei Reste zusammen Teil eines N-heterobicyclischen oder N-tricyclischen Restes mit 5 bis 9 Ringatomen pro Cyclus stehen, wobei die Cyclen weitere Heteroatome wie zum Beispiel Sauerstoff oder Schwefel enthalten können.

Beispiele für erfindungsgemäße Basen der allgemeinen Formel (IV) sind Triethylamin, Trimethylamin, Diisopropylethylamin, Tri-*n*-propylamin, Tri-*n*-butylamin, Tri-*n*-hexylamin, Tricyclohexylamin, *N-*Methyl-cyclohexylamin, *N*-Methyl-pyrrolidin, *N-*Methyl-piperidin, *N-*Ethylpiperidin, *N,N-*Dimethylanilin, *N*-Methyl-morpholin, Pyridin, 2-, 3-, 4-Picolin, 2-Methyl-5-ethyl-pyridin, 2,6-Lutidin, 2,4,6-Collidin, 4-Dimethylaminopyridin, Chinolin, Chinaldin, *N,N,N,N-*Tetramethylethyl-diamin, *N,N-*Dimethyl-1,4-diazacyclohexan, *N,N*-Di-ethyl-1,4-diazacyclohexan, 1,8-Bis-(Dimethylamino)naphthalin, Diazabicyclooctan (DABCO), Diazabicyclononan (DBN), Diazabicycloundecan (DBU), Alkylimidazol, wie Methylimidazol und Butylimidazol.

Beispiele für erfindungsgemäße Alkali- oder Erdalkalimetallhydroxyde, -hydrogencarbonate oder - carbonate sowie sonstige anorganische wässrige Basen sind Natriumhydroxid, Kaliumhydroxid, Lithiumhydroxid, Calciumhydroxid, Natriumcarbonat, Kaliumcarbonat, Natriumhydrogencarbonat und Kaliumhydrogencarbonat. Die anorganische Base wird gegebenenfalls als wässrige Lösung in einer Konzentration im Bereich von etwa 10 bis 40 Gew.-% eingesetzt.

Besonders bevorzugte Basen sind Triethylamin, Tributylamin, Pyridin, 2-, 3-, 4-Picolin, 2-Methyl-5-ethyl-pyridin, 2,6-Lutidin, Methylimidazol, Butylimidazol, Natriumhydroxid oder Kaliumhydroxid.

Das molare Verhältnis der Base zu eingesetztem 2,2-Difluor-1-halogenethan der Formel (I) kann beispielsweise im Bereich von etwa 10 bis 0,5 liegen. Vorzugsweise liegt es im Bereich von etwa 8 bis 1, besonders bevorzugt im Bereich von etwa 6 bis 1,1. Der Einsatz größerer Mengen an Base ist grundsätzlich möglich, jedoch unwirtschaftlich.

Das erfindungsgemäßen Verfahren kann weiterhin in Gegenwart eines Katalysator durchgeführt werden. Geeignete Katalysatoren sind solche, die die Umsetzung mit dem Amin der Formel (II) beschleunigen. Mischungen geeigneter Katalysatoren sind auch denkbar. Erfindungsgemäß geeignet sind beispielsweise Alkalibromide und -jodide (z.B. Natriumjodid, Kaliumjodid, Kaliumbromid); Ammoniumbromid und Ammoniumjodid; Tetraalkylammoniumbromide und -jodide, (z.B. Tetraethylammoniumjodid); bestimmte Phosphoniumhalogenide, wie Tetraalkyl- oder Tetraarylphosphoniumhalogenide (z.B. Hexadecyl-tri-butyl-phosphoniumbromid, Stearyltributylphosphoniumbromid, Tetrabutylphosphoniumbromid, Tetraoctylphosphoniumbromid, Tetraphenylphosphoniumchlorid und Tetraphenylphosphoniumbromid), Tetrakis(dimethylamino)phosphoniumbromid, Tetrakis(diethyl-amino)phosphoniumbromid, Tetrakis(dipropylamino)phosphoniumchlorid und Tetrakis(dipropyl-amino)phosphoniumbromid; sowie Bis(dimethylamino)[(1,3-dimethylimidazolidin-2-yliden)amino]methyliumbromid.

Im erfindungsgemäßen Verfahren werden als Katalysatoren bevorzugt Kaliumbromid, Natriumjodid, Kaliumjodid, Tetrabutylammoniumbromid oder Tetraphenylphosphoniumbromid, besonders bevorzugt Natrium- oder Kaliumjodid sowie Kaliumbromid eingesetzt.

Wird 2,2-Difluor-1-chlorethan als Verbindung (I) eingesetzt, so ist es besonders vorteilhaft, das erfindungsgemäßen Verfahren in Gegenwart eines Katalysators durchzuführen, denn die Reaktion verläuft dann schneller.

Im erfindungsgemäßen Verfahren wird der Katalysator, bezogen auf das eingesetzte 2,2-Difluor-1-halogenethan der Formel (I), in einer Konzentration von etwa 0,01 Gew.-% bis etwa 25 Gew.-% verwendet. Höhere Konzentrationen sind grundsätzlich möglich. Bevorzugt wird der Katalysator in einer Konzentration von etwa 0,2 Gew.-% bis etwa 25 Gew.-%, besonders bevorzugt von etwa 0,4 Gew.-% bis etwa 20 Gew.-%, ganz besonders bevorzugt von etwa 0,5 Gew.-% bis etwa 15 Gew.-% verwendet. Der Katalysator kann aber auch bevorzugt in einer Konzentration von etwa 0,05 Gew.-% bis etwa 3 Gew.-%, von etwa 0,1 Gew.-% bis etwa 10 Gew.-% oder von etwa 0,5 Gew.-% bis etwa 10 Gew.-% eingesetzt werden.

Sofern nichts anderes angegeben, wird unter dem Begriff "Alkyl", entweder in Alleinstellung oder aber in Kombination mit weiteren Begriffen, wie beispielsweise Halogenalkyl, im Rahmen der vorliegenden Erfindung ein Rest einer gesättigten, aliphatischen Kohlenwasserstoffgruppe mit 1 bis 12 Kohlenstoffatomen verstanden, die verzweigt oder unverzweigt sein kann. Beispiele für C₁-C₁₂-Alkylreste sind Methyl, Ethyl, n-Propyl, iso-Propyl, n-Butyl, iso-Butyl, sek.-Butyl, tert.-Butyl, n-Pentyl, iso-Pentyl, Neopentyl, tert.-Pentyl, 1-Methylbutyl, 2-Methylbutyl, 1-Ethylpropyl, 1,2-Dimethylpropyl, Hexyl, n-Heptyl, n-Octyl, n-Nonyl, n-Decyl, n-Undecyl und n-Dodecyl. Von diesen Alkylresten sind C₁-C₆-Alkylreste besonders bevorzugt. Insbesondere bevorzugt sind C₁-C₄-Alkylreste.

Sofern nichts anderes angegeben, wird unter dem Begriff "Aryl" erfindungsgemäß ein aromatischer Rest mit 6 bis 14 Kohlenstoffatomen, vorzugsweise Phenyl, verstanden.

Sofern nichts anderes angegeben, wird unter dem Begriff "Arylalkyl" eine Kombination von erfindungsgemäß definierten Resten "Aryl" und "Alkyl" verstanden, wobei der Rest im Allgemeinen über die Alkylgruppe gebunden wird, Beispiele hierfür sind Benzyl, Phenylethyl oder α-Methylbenzyl, wobei Benzyl besonders bevorzugt ist.

Im Rahmen der vorliegenden Erfindung werden unter durch Halogen substituierte Reste, beispielsweise Halogenalkyl, einfach oder mehrfach bis zur maximal möglichen Substituentenzahl halogenierte Reste verstanden. Bei mehrfacher Halogenierung können die Halogenatome gleich oder verschieden sein. Halogen steht dabei für Fluor, Chlor, Brom oder Jod.

Unter dem Begriff "Alkoxy", entweder in Alleinstellung oder aber in Kombination mit weiteren Begriffen, wie beispielsweise Halogenalkoxy, wird vorliegend ein Rest O-Alkyl verstanden, wobei der Begriff "Alkyl" die oben stehende Bedeutung aufweist.

Gegebenenfalls substituierte Reste können einfach oder mehrfach substituiert sein, wobei bei einer Mehrfachsubstitutionen die Substituenten gleich oder verschieden sein können.

Im erfindungsgemäßen Verfahren liegt das molare Verhältnis von 2,2-Difluor-1-halogenethan der allgemeinen Formel (I) zum eingesetzten Amin der allgemeinen Formel (II) im Bereich von etwa 1 : 1,5 bis etwa 20 : 1, bevorzugt im Bereich von etwa 1 : 1 bis etwa 10 : 1, besonders bevorzugt von etwa 1 : 1 bis etwa 3 : 1.

Das erfindungsgemäße Verfahren kann ohne oder mit Lösungsmittel durchgeführt werden. Wird eine Lösungsmittel eingesetzt, so wird es in einer solchen Menge verwendet, dass das Reaktionsgemisch während des ganzen Verfahrens gut rührbar ist. Vorteilhafterweise wird, bezogen auf das eingesetzte 2,2-Difluor-1-halogenethan der Formel (I), die 1- bis 50-fache Lösungsmittelmenge, bevorzugt die 2- bis 40-fache Lösungsmittelmenge, besonders bevorzugt die 2- bis 20-fache Lösungsmittelmenge verwendet.

Als Lösungsmittel zur Durchführung des erfindungsgemäßen Verfahrens kommen alle unter den Reaktionsbedingungen inerten organischen Lösungsmittel in Frage. Inerte Lösungsmittel sind solche, die unter den jeweilig gegebenen Bedingungen mit potentiellen Reaktionspartnern nicht oder nur in verschwindend geringem Maße reagieren. Unter Lösungsmittel werden erfindungsgemäß auch Gemische reiner Lösungsmittel verstanden.

Erfindungsgemäß geeignete Lösungsmittel sind insbesondere Alkohole (z.B. Methanol, Ethanol, Isopropanol, Butanol (d.h. n-Butanol, tert-Butanol, 2-Butanol), 2-(2-Ethoxyethoxy)-ethanol, Diethylenglykol); Ether (z.B. Ethylpropylether, Methyl-tert-butylether, *n*-Butylether, Anisol, Phenetol, Cyclohexylmethylether, Dimethylether, Diethylether, Dimethylglycol, Diphenylether, Dipropylether, Diisopropylether, Di-*n*-butylether, Diisobutylether, Diisoamylether, Ethylenglycoldimethylether, Isopropylethylether, Diethylenglykoldimethylether, Triethylenglykoldimethylether, Tetrahydrofuran, Dioxan, und Polyether des Ethylenoxids und/oder Propylenoxids); Verbindungen wie Tetrahydrothiophendioxid und Dimethylsulfoxid, Tetramethylensulfoxid, Dipropylsulfoxid, Benzylmethylsulfoxid, Diisobutylsulfoxid, Dibutylsulfoxid, Diisoamylsulfoxid; Sulfone wie Dimethyl-, Diethyl-, Dipropyl-, Dibutyl-, Diphenyl-, Dihexyl-, Methylethyl-, Ethylpropyl-, Ethylisobutyl- und Pentamethylensulfon; aliphatische, cycloaliphatische oder aromatische Kohlenwasserstoffe (z.B. Pentan, Hexan, Heptan, Oktan, Nonan wie die sogenannten "White Spirits" mit Komponenten mit Siedepunkten im Bereich beispielsweise von 40°C bis 250°C, Cymol, Benzinfraktionen innerhalb eines Siedeintervalls von 70°C bis 190°C, Cyclohexan, Methylcyclohexan, Petrolether, Ligroin, Octan, Benzol, Toluol, Chlorbenzol, Brombenzol, Xylol); Ester (z.B. Methyl-, Ethyl-, Butyl-, Isobutylacetat, Dimethyl-, Dibutyl-oder Ethylencarbonat, Propylencarbonat); Amide (z.B. Hexamethylphosphorsäuretriamid, Formamid, N,N-Dimethyl-acetamid, *N*-Methyl-formamid, *N,N-*Dimethyl-formamid, *N,N*-Dipropyl- formamid, *N*,*N-*Dibutyl-formamid, *N-*Methyl-pyrrolidon, *N-*Methyl-caprolactam, 1,3-Dimethyl-3,4,5,6-tetrahydro-2(1H)-pyrimidin, Octylpyrrolidon, Octylcaprolactam, 1,3-Dimethyl-2-imidazolindion, *N*-Formyl-piperidin, *N,N'-*1,4-Diformyl-piperazin) oder Gemische davon.

Im erfindungsgemäßen Verfahren werden als Lösungsmittel bevorzugt Alkohole, insbesondere *n*-Butanol, Amide, insbesondere *N*-Methyl-pyrrolidon oder 1,3-Dimethyl-2-imidazolindion, Ether, insbesondere Triethylenglykoldimethylether, sowie Dimethylsulfoxid oder Tetramethylensulfoxid oder Gemische davon eingesetzt.

Die erfindungsgemäße Umsetzung kann in einem weiten Temperaturbereich (z.B. im Bereich von 50°C bis 200°C) durchgeführt werden. Vorzugsweise wird die Umsetzung in einem Temperaturbereich von 70° bis 160°C durchgeführt.

Die Umsetzung wird grundsätzlich unter Eigendruck in einem druckstabilen geschlossenen Versuchsgefäß (Autoklav) durchgeführt. Der Druck während der Reaktion (d.h. der Eigendruck) ist abhängig von der verwendeten Reaktionstemperatur, dem verwendeten Lösungsmittel und dem verwendeten 2,2-Difluor-1-halogenethan. Ist eine Druckerhöhung gewünscht, so kann eine zusätzliche Druckerhöhung durch Zugabe bzw. Zuleiten eines Inertgases, wie Stickstoff oder Argon, durchgeführt werden.

Die Reaktionsdauer der Reaktion ist kurz und liegt im Bereich von etwa 0,5 bis etwa 16 Stunden. Eine längere Reaktionsdauer ist möglich, jedoch wirtschaftlich nicht sinnvoll.

Die Aufarbeitung des Reaktionsgemisches sowie die Aufreinigung kann z.B. durch Destillation des 2,2-Difluorethylamin-Derivats der Formel (III) durchgeführt werden oder über die entsprechenden Salze. Normalerweise wird die Reaktionsmischung auf Wasser gegossen und der pH Wert der Lösung auf 12 eingestellt. Durch Extraktion mit einem Lösungsmittel kann das 2,2-Difluorethylamin-Derivat der Formel (III) extrahiert werden und anschließend unter Normaldruck oder im Vakuum, vorzugsweise durch Destillation, isoliert werden.

Die Aufreinigung eines Salzes der 2,2-Difluorethylamin-derivate der allgemeinen Formel (III), beispielsweise Salze organischer oder anorganischer Säuren (z.B. Hydrochloride oder Acetate), erfolgt bevorzugt durch Kristallisation. Wasserlösliche Salze können durch Extraktion der wässrigen Lösungen gereinigt werden. Das Amin kann dann schließlich durch Umsetzung mit organischen oder anorganischen Basen aus seinen Salzen freigesetzt werden. Bevorzugte Basen sind NaHCO₃, Na₂CO oder NaOH.

Die vorliegende Erfindung wird anhand der nachfolgenden Beispiele näher erläutert, ohne die Erfindung dabei auf diese einzuschränken.

### Beispiel 1

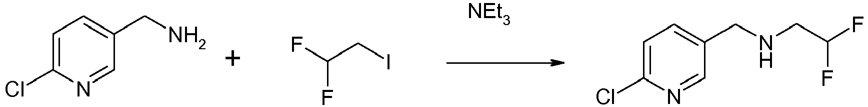

26,3 g (0,135 mol) 2,2-Difluor-1-jodethan, 10 g (0,067 mol) 1-(6-Chlorpyridin-3-yl)methanamin und 8,2 g Triethylamin werden mit 31 g *N*-Methyl-pyrrolidon vorgelegt. Die Mischung wird 50 Minuten auf 100 °C erhitzt und anschließend wieder auf 80°C abgekühlt. Das *N*-Methyl-pyrrolidon wird im Vakuum bei 80°C abdestilliert und die Reaktionsmischung wird auf 50 ml Wasser gegossen. Mit 3 ml 45%iger Natronlauge wird auf pH 12 eingestellt und dann zweimal mit 30 ml Toluol extrahiert. Das Produkt wird anschließend im Vakuum feindestilliert. Man erhält 11, 1 g N-[(6-Chlorpyridin-3-yl)methyl]-2,2-difluorethanamin (entspricht 79,5 % Ausbeute).
NMR(d-DMSO): 1H(s, 8,35 ppm); 1H (dd,7,82 ppm ); 1H (d,7,46 ppm ); 1 H (tt, 6,02 ppm); 2 H (s, 3,8 ppm); 2 H (td, 2,9 ppm)

### Beispiel 2

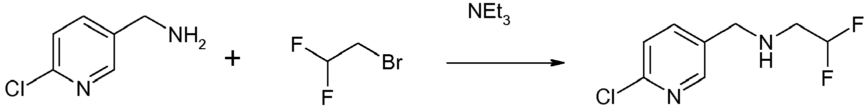

19,9 g (0,135 mol) 2,2-Difluor-1-bromethan, 10 g (0,067 mol) 1-(6-Chlorpyridin-3-yl)methanamin und 8,2 g Triethylamin werden mit 31 g N-Methyl-pyrrolidon vorgelegt. Die Mischung wird 2 Stunden auf 100 °C erhitzt und anschließend wieder auf 80°C abgekühlt. Das *N*-Methyl-pyrrolidon wird im Vakuum bei 80°C abdestilliert und die Reaktionsmischung wird auf 50 ml Wasser gegossen. Mit 2 ml 45%iger Natronlauge wird auf pH 12 eingestellt und dann zweimal mit 30 ml Toluol extrahiert. Das Produkt wird anschließend im Vakuum feindestilliert. Man erhält 11,5 g N-[(6-Chlorpyridin-3-yl)methyl]-2,2-difluorethanamin (entspricht 83,1 % Ausbeute).
NMR(d-DMSO): 1H(s, 8,35 ppm); 1H (dd,7,82 ppm ); 1H (d,7,46 ppm ); 1 H (tt, 6,02 ppm); 2 H (s, 3,8 ppm); 2 H (td, 2,9 ppm)

### Beispiel 3

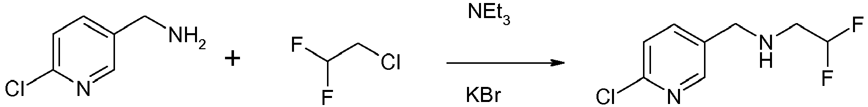

13,7 g (0,135 mol) 2,2-Difluor-1-chlorethan, 10 g (0,067 mol) 1-(6-Chlorpyridin-3-yl)methanamin und 8,2 g Triethylamin werden mit 31 g *N*-Methyl-pyrrolidon vorgelegt. Zusätzlich werden 4 g (0,033 mol) Kaliumbromid zugesetzt. Die Mischung wird 16 Stunden im Autoklav unter Eigendruck auf 120 °C erhitzt und anschließend wieder auf 80°C abgekühlt. Das *N*-Methyl-pyrrolidon wird im Vakuum bei 80°C abdestilliert und die Reaktionsmischung wird auf 20 ml 32 %ige Salzsäure gegossen. Die Mischung wird im Vakuum zur Trockene eingeengt und dann mit 10 ml 45%iger NaOH auf pH von 12 eingestellt. Dreimal wird mit 30 ml Toluol extrahiert und die organischen Phasen werden im Vakuum destilliert. Man erhält 9,8 g N-[(6-Chlorpyridin-3-yl)methyl]-2,2-difluorethanamin (entspricht 71 % Ausbeute).
NMR(d-DMSO): 1H(s, 8,35 ppm); 1H (dd,7,82 ppm ); 1H (d,7,46 ppm ); 1 H (tt, 6,02 ppm); 2 H (s, 3,8 ppm); 2 H (td, 2,9 ppm)

## Patentansprüche

1. Verfahren zur Herstellung von 2,2-Difluorethylamin-Derivaten der allgemeinen Formel (III) 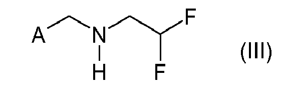 in der
A für einen gegebenenfalls substituierten Heterocyclus steht, der ausgewählt ist aus einer Gruppe bestehend aus Pyrid-2-yl, Pyrid-4-yl und Pyrid-3-yl, die gegebenenfalls in 6-Position substituiert sind durch Fluor, Chlor, Brom, Methyl, Trifluormethyl oder Trifluormethoxy, sowie Pyridazin-3-yl, welches gegebenenfalls in 6-Position substituiert ist durch Chlor oder Methyl sowie Pyrazin-3-yl, 2-Chlor-pyrazin-5-yl und 1,3-Thiazol-5-yl, wobei 1,3-Thiazol-5-yl gegebenenfalls in 2-Position substituiert ist durch Chlor oder Methyl, sowie Pyrimidinyl, Pyrazolyl, Thiophenyl, Oxazolyl, Isoxazolyl, 1,2,4-Oxadiazolyl, Isothiazolyl, 1,2,4-Triazolyl und 1,2,5-Thiadiazolyl, die gegebenenfalls durch Fluor, Chlor, Brom, Cyano, Nitro, gegebenenfalls durch Fluor und/oder Chlor substituiertes C₁-C₄-Alkyl, gegebenenfalls durch Fluor und/oder Chlor substituiertes C₁-C₃-Alkylthio, oder gegebenenfalls durch Fluor und/oder Chlor substituiertes C₁-C₃-Alkylsulfonyl substituiert sind und einem Pyrid-3-yl der folgenden Formel 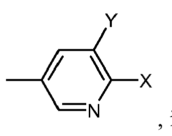 in der
X für Halogen, C₁-C₁₂-Alkyl oder C₁-C₁₂-Halogenalkyl steht und
Y für Halogen, C₁-C₁₂-Alkyl, C₁-C₁₂-Halogenalkyl, C₁-C₁₂-Halogenalkoxy, Azido oder Cyan steht,
in dem eine 2,2-Difluorethyl-1-halogenethan Verbindung der allgemeinen Formel (I) 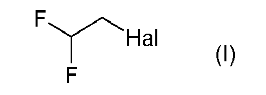 in der Hal für Cl, Br oder Jod steht mit einem Amin der allgemeinen Formel (II) 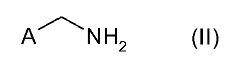 in der A die vorgenannten Bedeutungen hat,
gegebenenfalls in Gegenwart einer Base umgesetzt wird.

2. Verfahren nach Anspruch 1, wobei das molare Verhältnis von 2,2-Difluor-1-halogenethan der allgemeinen Formel (I) zum eingesetzten Amin der allgemeinen Formel (II) im Bereich von 1 : 1,5 bis 20 : 1 liegt.

3. Verfahren nach Anspruch 1 oder 2, wobei die Base ausgewählt ist unter tertiären Stickstoffbasen, anorganische wässrige Basen, und Alkali- oder Erdalkalimetallhydroxyde, -hydrogencarbonate oder -carbonate.

4. Verfahren nach Anspruch 3, wobei die Base ausgewählt ist unter substituierten oder unsubstituierten Pyridinen, substituierten oder unsubstituierten Chinolinen und tertiären Aminen der allgemeinen Formel (IV)
NR¹R²R³ (IV)
worin
R¹, R² und R³ unabhängig voneinander für C₁₋₁₂-Alkyl, C₆₋₁₈-Aryl, C₇₋₁₉-Alkylaryl- oder C₇₋₁₉-Arylalkyl stehen, oder in der zwei der Reste zusammen für einen 5- bis 8- gliedrigen Stickstoff enthaltenden Heterocylus stehen, oder in der alle drei Reste zusammen Teil eines N-heterobicyclischen oder N-tricyclischen Restes mit 5 bis 9 Ringatomen pro Cyclus stehen, wobei die Cyclen weitere Heteroatome wie zum Beispiel Sauerstoff oder Schwefel enthalten können.

5. Verfahren nach Anspruch 4, wobei die Base ausgewählt ist unter Triethylamin, Trimethylamin, Diisopropylethylamin, Tri-*n*-propylamin, Tri-*n*-butylamin, Tri-*n*-hexylamin, Tricyclohexylamin, *N-*Methyl-cyclohexylamin, *N*-Methyl-pyrrolidin, *N-*Methyl-piperidin, *N-*Ethylpiperidin, *N*,*N-*Dimethylanilin, *N*-Methyl-morpholin, Pyridin, 2-, 3-, 4-Picolin, 2-Methyl-5-ethyl-pyridin, 2,6-Lutidin, 2,4,6-Collidin, 4-Dimethylaminopyridin, Chinolin, Chinaldin, *N,N,N,N-*Tetramethylethyl-diamin, *N,N*-Dimethyl-1,4-diazacyclohexan, *N,N-*Di-ethyl-1,4-diazacyclohexan, 1,8-Bis-(Dimethyl-amino)naphthalin, Diazabicyclooctan, Diazabicyclononan, Diazabicycloundecan, Methylimidazol und Butylimidazol, Natriumhydroxid, Kaliumhydroxid, Lithiumhydroxid, Calciumhydroxid, Natriumcarbonat, Kaliumcarbonat, Natriumhydrogencarbonat und Kaliumhydrogencarbonat.

6. Verfahren nach einem der Ansprüche 1 bis 5, wobei die Umsetzung in Gegenwart eines Katalysators und gegebenenfalls in Gegenwart einer Base stattfindet.

7. Verfahren nach Anspruch 6, wobei der Katalysator ausgewählt ist unter Alkalibromide und - jodide, Ammoniumbromid und Ammoniumjodid, Tetraalkylammoniumbromide und -jodide, Tetraalkyl- oder Tetraarylphosphoniumhalogenide, Tetrakis(dimethylamino)phosphoniumbromid, Tetrakis(diethylamino)phosphoniumbromid, Tetrakis(dipropylamino)phosphoniumchlorid und Tetrakis(dipropylamino)phosphoniumbromid, und Bis(dimethylamino)[(1,3-dimethylimidazolidin-2-yliden)amino]methyliumbromid.

8. Verfahren nach Anspruch 6, wobei der Katalysator ausgewählt ist unter Kaliumbromid, Natriumjodid, Kaliumjodid, Tetrabutylammoniumbromid und Tetraphenylphosphoniumbromid.

## Claims

1. Process for preparing 2,2-difluoroethylamine derivatives of the general formula (III) 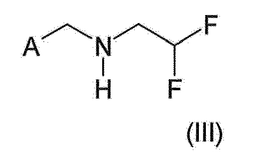 in which
A is an optionally substituted heterocycle which is selected from a group consisting of pyrid-2-yl, pyrid-4-yl and pyrid-3-yl which are optionally 6-substituted by fluorine, chlorine, bromine, methyl, trifluoromethyl or trifluoromethoxy, and pyridazin-3-yl, which is optionally 6-substituted by chlorine or methyl, and pyrazin-3-yl, 2-chloropyrazin-5-yl and 1,3-thiazol-5-yl, where 1,3-thiazol-5-yl is optionally 2-substituted by chlorine or methyl, and pyrimidinyl, pyrazolyl, thiophenyl, oxazolyl, isoxazolyl, 1,2,4-oxadiazolyl, isothiazolyl, 1,2,4-triazolyl and 1,2,5-thiadiazolyl which are optionally substituted by fluorine, chlorine, bromine, cyano, nitro, optionally fluorine- and/or chlorine-substituted C₁-C₄-alkyl, optionally fluorine- and/or chlorine-substituted C₁-C₃-alkylthio, or optionally fluorine- and/or chlorine-substituted C₁-C₃-alkylsulphonyl, and a pyrid-3-yl of the following formula 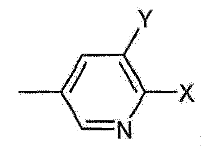 in which
X is halogen, C₁-C₁₂-alkyl or C₁-C₁₂-haloalkyl
and
Y is halogen, C₁-C₁₂-alkyl, C₁-C₁₂-haloalkyl, C₁-C₁₂-haloalkoxy, azido or cyano,
by reacting a 2,2-difluoroethyl-1-haloethane compound of the general formula (I) 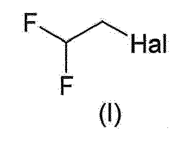 in which Hal is Cl, Br or iodine with an amine of the general formula (II) 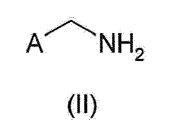 in which A is as defined above,
optionally in the presence of a base.

2. Process according to Claim 1, wherein the molar ratio of 2,2-difluoro-1-haloethane of the general formula (I) to the amine of the general formula (II) used is in the range from 1:1.5 to 20:1.

3. Process according to Claim 1 or 2, wherein the base is selected from tertiary nitrogen bases, inorganic aqueous bases, and alkali metal or alkaline earth metal hydroxides, hydrogencarbonates or carbonates.

4. Process according to Claim 3, wherein the base is selected from substituted and unsubstituted pyridines, substituted and unsubstituted quinolines and tertiary amines of the general formula (IV)
NR¹R²R³ (IV)
in which
R¹, R² and R³ are each independently C₁₋₁₂-alkyl, C₆₋₁₈-aryl, C₇₋₁₉-alkylaryl- or C₇₋₁₉-arylalkyl, or in which two of the radicals together are a 5- to 8-membered nitrogen-containing heterocycle, or in which all three radicals together are part of an N-heterobicyclic or N-tricyclic radical having 5 to 9 ring atoms per cycle, where the cycles may contain further heteroatoms, for example oxygen or sulphur.

5. Process according to Claim 4, wherein the base is selected from triethylamine, trimethylamine, diisopropylethylamine, tri-*n*-propylamine, tri-*n-*butylamine, tri-*n*-hexylamine, tricyclohexylamine, *N*-methylcyclohexylamine, N-methylpyrrolidine, N-methylpiperidine, *N*-ethylpiperidine, *N,N-*dimethylaniline, N-methylmorpholine, pyridine, 2-, 3-, 4-picoline, 2-methyl-5-ethylpyridine, 2,6-lutidine, 2,4,6-collidine, 4-dimethylaminopyridin, quinoline, quinaldine, *N,N,N,N*-tetramethyl-ethyldiamine, *N,N*-dimethyl-1,4-diazacyclohexane, *N,N*-diethyl-1,4-diazacyclohexane, 1,8-bis(dimethylamino)naphthalene, diazabicyclooctane, diazabicyclononane, diazabicycloundecane, methylimidazole and butylimidazole, sodium hydroxide, potassium hydroxide, lithium hydroxide, calcium hydroxide, sodium carbonate, potassium carbonate, sodium hydrogencarbonate and potassium hydrogencarbonate.

6. Process according to any of Claims 1 to 5, wherein the reaction takes place in the presence of a catalyst and optionally in the presence of a base.

7. Process according to Claim 6, wherein the catalyst is selected from alkali metal bromides and iodides, ammonium bromide and ammonium iodide, tetraalkylammonium bromides and iodides, tetraalkyl- or tetraarylphosphonium halides, tetrakis(dimethylamino)phosphonium bromide, tetrakis(diethylamino)phosphonium bromide, tetrakis(dipropylamino)phosphonium chloride and tetrakis(dipropylamino)phosphonium bromide, and bis(dimethylamino)[(1,3-dimethylimidazolidin-2-ylidene)amino]methylium bromide.

8. Process according to Claim 6, wherein the catalyst is selected from potassium bromide, sodium iodide, potassium iodide, tetrabutylammonium bromide and tetraphenylphosphonium bromide.

## Revendications

1. Procédé de fabrication de dérivés de 2,2-difluoroéthylamine de formule générale (III) 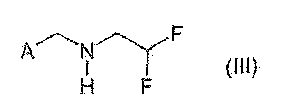 dans laquelle
A représente un hétérocycle éventuellement substitué, qui est choisi dans un groupe constitué par pyrid-2-yle, pyrid-4-yle et pyrid-3-yle, qui sont éventuellement substitués en position 6 par fluor, chlore, brome, méthyle, trifluorométhyle ou trifluorométhoxy, ainsi que pyridazin-3-yle, qui est éventuellement substitué en position 6 par chlore ou méthyle, ainsi que pyrazin-3-yle, 2-chloro-pyrazin-5-yle et 1,3-thiazol-5-yle, 1,3-thiazol-5-yle étant éventuellement substitué en position 2 par chlore ou méthyle, ainsi que pyrimidinyle, pyrazolyle, thiophényle, oxazolyle, isoxazolyle, 1,2,4-oxadiazolyle, isothiazolyle, 1,2,4-triazolyle et 1,2,5-thiadiazolyle, qui sont éventuellement substitués par fluor, chlore, brome, cyano, nitro, alkyle en C₁-C₄ éventuellement substitué par fluor et/ou chlore, alkylthio en C₁-C₃ éventuellement substitué par fluor et/ou chlore, ou alkylsulfonyle en C₁-C₃ éventuellement substitué par fluor et/ou chlore, et un pyrid-3-yle de formule générale 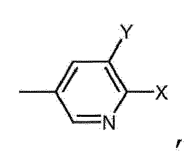 dans laquelle
X représente halogène, alkyle en C₁-C₁₂ ou halogénoalkyle en C₁-C₁₂, et
Y représente halogène, alkyle en C₁-C₁₂, halogénoalkyle en C₁-C₁₂, halogénoalcoxy en C₁-C₁₂, azido ou cyano,
selon lequel un composé de 2,2-difluoroéthyl-1-halogénoéthane de formule générale (I) 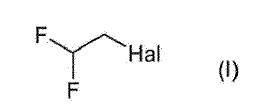 dans laquelle Hal représente Cl, Br ou iode,
est mis en réaction avec une amine de formule générale (II) 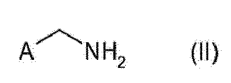 dans laquelle A a les significations susmentionnées, éventuellement en présence d'une base.

2. Procédé selon la revendication 1, dans lequel le rapport molaire entre le 2,2-difluoro-1-halogénoéthane de formule générale (I) et l'amine utilisée de formule générale (II) se situe dans la plage allant de 1:1,5 à 20:1.

3. Procédé selon la revendication 1 ou 2, dans lequel la base est choisie parmi les bases azotées tertiaires, les bases aqueuses inorganiques, et les hydroxydes, hydrogénocarbonates ou carbonates de métaux alcalins ou alcalino-terreux.

4. Procédé selon la revendication 3, dans lequel la base est choisie parmi les pyridines substituées ou non substituées, les quinolines substituées ou non substituées et les amines tertiaires de formule générale (IV)
NR¹R²R³ (IV)
dans laquelle
R¹, R² et R³ représentent indépendamment les uns des autres alkyle en C₁₋₁₂, aryle en C₆₋₁₈, alkylaryle en C₇₋₁₉ ou arylalkyle en C₇₋₁₉, ou deux des radicaux représentent ensemble un hétérocycle azoté de 5 à 8 éléments, ou les trois radicaux représentent ensemble une partie d'un radical N-hétérobicyclique ou N-tricyclique contenant 5 à 9 atomes de cycle par cycle, les cycles pouvant contenir des hétéroatomes supplémentaires tels que par exemple l'oxygène ou le soufre.

5. Procédé selon la revendication 4, dans lequel la base est choisie parmi la triéthylamine, la triméthylamine, la diisopropyléthylamine, la tri-*n-*propylamine, la tri-*n*-butylamine, la tri-*n*-hexylamine, la tricyclohexylamine, la *N*-méthyl-cyclohexylamine, la *N*-méthyl-pyrrolidine, la N-méthyl-pipéridine, la N-éthylpipéridin, la *N,N*-diméthylaniline, la *N*-méthyl-morpholine, la pyridine, la 2-, 3-, 4-picoline, la 2-méthyl-5-éthyl-pyridine, la 2,6-lutidine, la 2,4,6-collidine, la 4-diméthylaminopyridine, la quinoline, la quinaldine, la *N,N,N,N*-tétraméthyléthyl-diamine, le *N,N*-diméthyl-1,4-diazacyclohexane, le *N,N*-diéthyl-1,4-diazacyclohexane, la 1,8-bis-(diméthyl-amino)naphtaline, le diazabicyclooctane, le diazabicyclononane, le diazabicycloundécane, le méthylimidazole et le butylimidazole, l'hydroxyde de sodium, l'hydroxyde de potassium, l'hydroxyde de lithium, l'hydroxyde de calcium, le carbonate de sodium, le carbonate de potassium, l'hydrogénocarbonate de sodium et l'hydrogénocarbonate de potassium.

6. Procédé selon l'une quelconque des revendications 1 à 5, dans lequel la réaction a lieu en présence d'un catalyseur et éventuellement en présence d'une base.

7. Procédé selon la revendication 6, dans lequel le catalyseur est choisi parmi les bromures et iodures alcalins, le bromure d'ammonium et l'iodure d'ammonium, les bromures et iodures de tétraalkylammonium, les halogénures de tétraalkyl- ou tétraarylphosphonium, le bromure de tétrakis(diméthylamino)phosphonium, le bromure de tétrakis(diéthylamino)phosphonium, le chlorure de tétrakis(dipropylamino)phosphonium et le bromure de tétrakis(dipropylamino)phosphonium, et le bromure de bis(diméthylamino)[(1,3-diméthylimidazolidin-2-ylidène)amino]méthylium.

8. Procédé selon la revendication 6, dans lequel le catalyseur est choisi parmi le bromure de potassium, l'iodure de sodium, l'iodure de potassium, le bromure de tétrabutylammonium et le bromure de tétraphénylphosphonium.
